# EUROPEAN PATENT APPLICATION

(11) **EP 1 552 836 A1**
(43) Date of publication of application: **13.07.2005**
(21) Application number: 03758700.3
(22) Date of filing: 30.09.2003
(51) Int. Cl.: A61K 31/495, A61K 31/454, A61K 31/496, A61P 25/00, A61P 25/14, A61P 25/16, A61P 25/28, C07D 295/14

(54) **ANTI-NEURODEGENERATIVE AGENTS**

(30) Priority: 30.09.2002 JP 2002286400
(71) Applicant: Ikeda, Joh-E, Meguro-ku, Tokyo 153-0051 (JP)
(72) Inventor: IKEDA, Joh-E, Meguro-ku, Tokyo 153-0051 (JP); OKADA, Yoshinori c/o Tokai University School, Isehara-shi, Kanagawa 259-1143 (JP); SAKAI, Harumi c/o Tokai University The, Isehara-shi, Kanagawa 259-1143 (JP); OSUGA, Hitoshi c/o Tokai University The Institute, Isehara-shi, Kanagawa 259-1143 (JP)
(74) Representative: Grünecker, Kinkeldey, Stockmair & Schwanhäusser
(86) International application number: PCT/JP2003/012540
(87) International publication number: WO 2004/028540

(57) **Abstract**

The present invention provides pharmaceutical agents useful for treatment and prevention of neurodegenerative diseases such as amyotrophic lateral sclerosis (ALS), spinal muscular atrophy (SMA), Huntington's disease, Parkinson's disease, Alzheimer's disease, dementia after cerebral vascular disorder, dementia accompanied by other neuronal degeneration. The agents comprise one, two or more compounds selected from the group consisting of 3-[4-(4-chlorophenyl) piperazin-1-yl]methyl]-1H-pyrrolo [2,3-b] pyridine or salts thereof, 5-(4-chlorophenyl)-4-methyl-3-(1-(2-phenylethyl) piperidin-4-yl) isoxazole or salts thereof, 3-(4-chlorophenyl)-4-methyl-5-(1-(2-phenylethyl) piperidin-4-yl) isoxazole or salts thereof, and N-methyl-4-(2-cyanophenyl) piperazinyl-3-methylbenzamine or salts thereof.

## Description

### Technical Field

The present invention relates to anti-neurodegenerative agents which are useful in treating and preventing neurodegenerative diseases such as amyotrophic lateral sclerosis (ALS), spinal muscular atrophy (SMA), Huntington's disease, Parkinson's disease, Alzheimer's disease, dementia after cerebral vascular disorders, and dementia accompanied by other neuronal degeneration.

### Background Art

The group of diseases involving neural cell group degeneration, such as amyotrophic lateral sclerosis (ALS), spinal muscular atrophy (SMA), Huntington's disease, Parkinson's disease, Alzheimer's disease, dementia caused by cerebral vascular disorders, and dementia accompanied by other neuronal degeneration, is generally referred to as neurodegenerative diseases. Fundamental methods of treatment have not been established for most neurodegenerative diseases, and thus treatment methods are being sought.

One approach to treating neurodegenerative diseases is considered to be the administration of factors that suppress neural cell degeneration. Administration of factors that suppress neurodegeneration is expected to be effective in treating and preventing these diseases. However, as yet virtually no such factors have been found to be actually applicable as effective therapeutic drugs.

As the factors that suppress neural cell degeneration, for example, certain dopamine receptor agonists are known to possibly have such a suppression function. However, the causal relationship between dopamine antagonists and the suppression of neural cell degeneration is unclear. Moreover, not all dopamine receptor agonists have this effect. In addition, to obtain substances effective as therapeutic drugs, the discovery of other classes of substances that can be used as anti-neurodegenerative drugs is also being sought.

### Disclosure of the Invention

The present inventors isolated the neuronal apoptosis inhibitory protein (NAIP) gene, a causative gene of the familial hereditary disease SMA, from the human chromosome 5q13.1 region (Roy *et al.,* Cell 80: 167-178, 1995). The present inventors also identified the entire amino acid sequence of NAIP, and isolated cDNAs encoding NAIP (Unexamined Published Japanese Patent Application No. (JP-A) Hei 11-116599). Moreover, while searching for compounds that increase NAIP production, the present inventors unexpectedly discovered that certain dopamine receptor antagonists, as well as certain dopamine receptor agonists, increased NAIP production. Furthermore, the present inventors discovered that these antagonists were indeed able to suppress neurodegeneration, thus completing the present invention.

Specifically, the present invention provides anti-neurodegenerative agents comprising one, two or more compounds selected from the group consisting of 3-[4-(4-chlorophenyl) piperazin-1-yl] methyl]-1H-pyrrolo [2,3-b] pyridine (hereinafter referred to as "compound 1") or its salts, 5-(4-chlorophenyl)-4-methyl-3-(1-(2-phenylethyl) piperidin-4-yl) isoxazole (hereinafter referred to as "compound 2") or its salts, 3-(4-chlorophenyl)-4-methyl-5-(1-(2-phenylethyl) piperidin-4-yl) isoxazole (hereinafter referred to as "compound 3") or its salts, and N-methyl-4-(2-cyanophenyl) piperazinyl-3-methylbenzamine (hereinafter referred to as "compound 4") or its salts.

Moreover, the present invention provides methods for producing anti-neurodegenerative agents using one, two or more compounds selected from the group consisting of 3-[4-(4-chlorophenyl) piperazin-1-yl] methyl]-1H-pyrrolo [2,3-b] pyridine or its salts, 5-(4-chlorophenyl)-4-methyl-3-(1-(2-phenylethyl) piperidin-4-yl) isoxazole or its salts, 3-(4-chlorophenyl)-4-methyl-5-(1-(2-phenylethyl) piperidin-4-yl) isoxazole or its salts, and N-methyl-4-(2-cyanophenyl) piperazinyl-3-methylbenzamine or its salts.

Moreover, the present invention provides methods for treating neurodegenerative diseases that comprise administering one, two or more compounds selected from the group consisting of 3-[4-(4-chlorophenyl) piperazin-1-yl]methyl]-1H-pyrrolo[2,3-b] pyridine or its salts, 5-(4-chlorophenyl)-4-methyl-3-(1-(2-phenylethyl) piperidin-4-yl) isoxazole or its salts, 3-(4-chlorophenyl)-4-methyl-5-(1-(2-phenylethyl) piperidin-4-yl) isoxazole or its salts, and N-methyl-4-(2-cyanophenyl) piperazinyl-3-methylbenzamine or its salts to a patient who has a neurodegenerative disease.

In the present invention, "neurodegenerative disease" means a disorder involving degeneration of the central nerve cells, such as amyotrophic lateral sclerosis (ALS), spinal muscular atrophy (SMA), Huntington's disease, Parkinson's disease, Alzheimer's disease, dementia after cerebral vascular disorder, and dementia accompanied by other neuronal degeneration. "Patients" mean human and non-human mammals (particularly useful mammals such as livestock and pets) that develop a neurodegenerative disease.

Other terms and concepts used in the present invention are defined in detail in this detailed description of the invention and in the Examples. Based on known literature and such, those skilled in the art could easily and precisely conduct the various techniques performed to carry out the present invention, except for techniques whose sources are specifically indicated. For example, formulations of the pharmaceutical agents in the present invention are described in Remington's Pharmaceutical Sciences, 18^{th} Edition, ed. A. Gennaro, Mack Publishing Co., Easton, PA, 1990, and such. Genetic engineering and molecular biology techniques are described in Sambrook and Maniatis, in Molecular Cloning-A Laboratory Manual, Cold Spring Harbor Laboratory Press, New York, 1989; Ausubel, F.M*. et al.,* Current Protocols in Molecular Biology, John Wiley & Sons, New York, N.Y, 1995, and such.

### Brief Description of Drawings

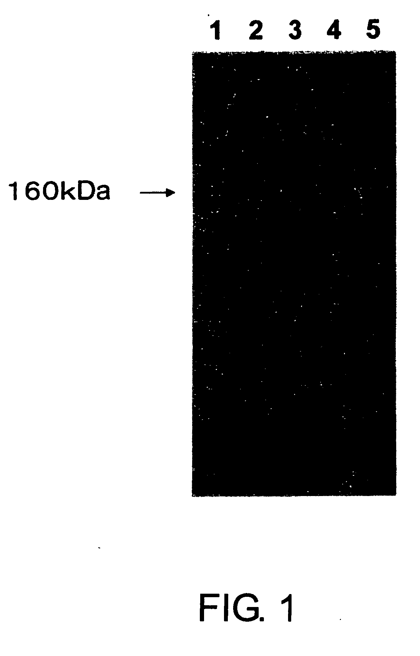

Fig. 1 is an electrophoretogram indicating changes in NAIP production due to compound 1 (Example 2). Lanes 1 to 5 in the figure show the results of electrophoresis of the samples indicated below.
   Lane 1: sample treated with a low concentration of retinoic acid and no candidate compound.
   Lane 2: sample treated with a low concentration of retinoic acid followed by addition of a candidate compound.
   Lane 3: sample treated with a high concentration of retinoic acid and no candidate compound.
   Lane 4: sample treated with a high concentration of retinoic acid followed by addition of a candidate compound.
   Lane 5: THP-1 cells
Fig. 2 is a graph showing the cell-death suppressing effect of compound 1 when menadione was used as a cell-death inducing agent (Example 3).
Fig. 3 is a graph showing the cell-death suppressing effect of compound 1 when α-naphthoquinone was used as a cell-death inducing agent (Example 3).
Fig. 4 is a graph showing the cell-death suppressing effect of compound 1 when 2, 3-dimethoxy-1, 4-naphthoquinone was used as a cell-death inducing agent (Example 3).
Fig. 5 is a graph showing the cell-death suppressing effect of compound 1 when menadione was used as a cell-death inducing agent (Example 4).
Fig. 6 is a graph showing the cell-death suppressing effect of compound 1 when menadione was used as a cell-death inducing agent (Example 5).
Fig. 7 is a microscope image of a tissue cross-section of the hippocampus CA1 region of a control Mongolian gerbil, in which compound administration and occlusion were not conducted (Example 6).
Fig. 8 is a microscope image of a tissue cross-section of the hippocampus CA1 region of a control Mongolian gerbil in which the same procedures as Examples 6-1 to 6-3 were conducted, except that the compound was not administered (Example 6).
Fig. 9 is a microscope image of a tissue cross-section of the hippocampus CA1 region of a Mongolian gerbil (Example 6-1).
Fig. 10 is a microscope image of a tissue cross-section of the hippocampus CA1 region of a Mongolian gerbil (Example 6-2).
Fig. 11 is a microscope image of a tissue cross-section of the hippocampus CA1 region of a Mongolian gerbil (Example 6-3).
Fig. 12 is a microscope image of a tissue cross-section of the hippocampus CA1 region of a control Mongolian gerbil, in which the same procedure as in Example 6-4 was conducted except that a compound was not administered (Example 6). The image is of a higher magnification than Fig. 8.
Fig. 13 is a microscope image of a tissue cross-section of the CA1 region of the hippocampus of a Mongolian gerbil (Example 6-4). The image is of the same magnification as Fig. 12.
   Table 1 shows the results of using various candidate compounds to conduct the tests of Example 2.
   Table 2 shows the results of testing the therapeutic effect of administering compound 1 to ALS model mice (Example 7).

### Best Mode for Carrying out the Invention

The anti-neurodegenerative drugs of the present invention comprise one, two or more compounds selected from a group consisting of compound 1 or its salts, compound 2 or its salts, compound 3 or its salts, and compound 4 or its salts as active ingredients.

Compounds 1 to 4 have the following structural formulas (1) to (4), respectively.

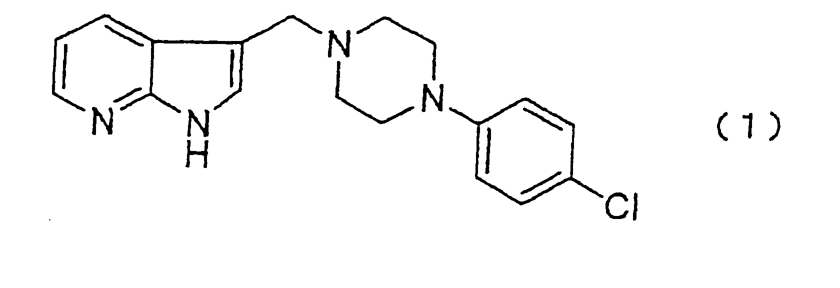

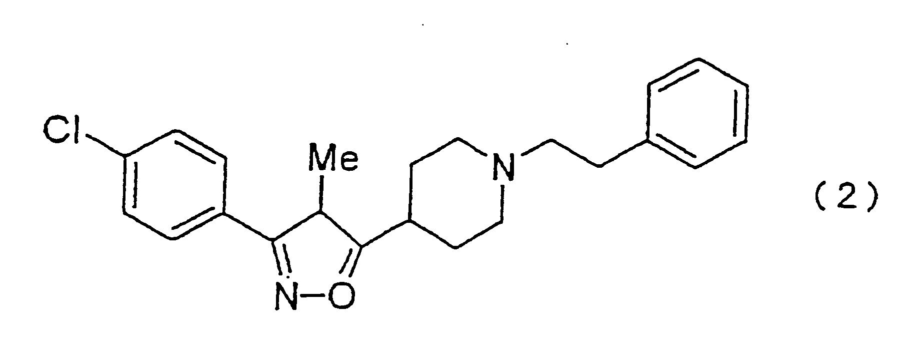

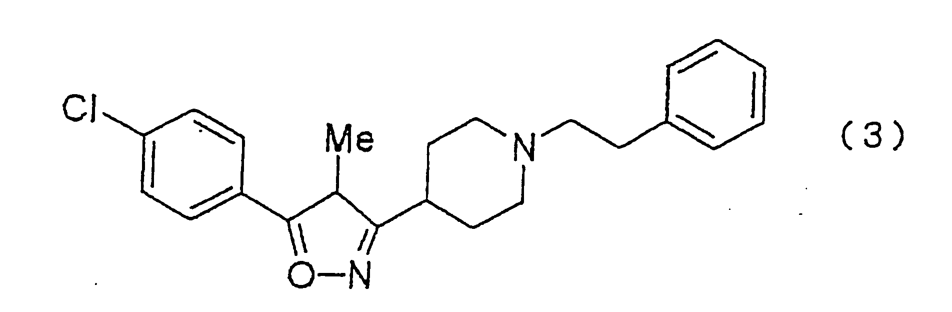

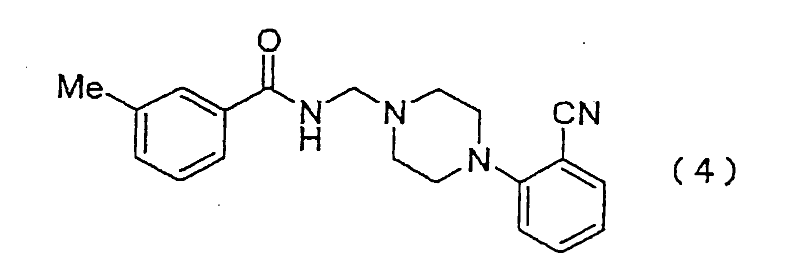

Compounds 1 to 3 are already known as dopamine D4 antagonists; compound 4 is already known as an agonist. They are commercially available from, for example, Tocris Cookson Ltd. (England).

Examples of the salts of compounds 1 to 4 include acids (inorganic or organic acids) addition salts, such as hydrochloride, hydrobromide, sulfate, nitrate, acetate, benzoate, maleate, fumarate, succinate, tartrate, citrate, oxalate, methanesulfonate, toluenesulfonate, aspartate, and glutamate. More specifically, for example, salts of compound 1 may be trihydrochloride, salts of compounds 2 and 3 may be monohydrochloride, and salts of compound 4 may be maleate.

The anti-neurodegenerative agents of the present invention can be the above described active ingredients alone. However, it is preferable to formulate them by mixing with pharmaceutically acceptable carriers, according to the symptoms and administration methods of the pharmaceutical agents. Specifically, the pharmaceutical agents of the present invention can be mixed with carriers to obtain dosage forms suitable for oral or parenteral administration.

Parenteral administration includes local infusion, intraperitoneal administration, selective intravenous infusion, intravenous injection, subcutaneous injection, organ perfusate infusion, rectal administration, and such. For example, the carriers used for formulation of injectables include sterile water, salt solution, glucose solution, or a mixture of salt solution and glucose, and such. Furthermore, pharmaceutical adjuvants such as buffers, pH controlling agents (disodium hydrogenphosphate, citric acid, and such), isotonizing agents (sodium chloride, glucose, and such), preservatives (methyl paraoxybenzoate, propyl p-hydroxybenzoate, and such), and such can also be comprised. The pharmaceutical agents formulated as above can be sterilized by filtration using sterilizing filters, by mixing the composition with disinfectants, or by irradiating or heating the composition. Alternatively, the agents can be formulated in a powder condition and can be mixed with an above described liquid carrier to prepare an injection solution at the time of use.

The orally administered agents can be formulated into a dosage form suitable for gastrointestinal absorption (for example, tablets, capsules, granules, micro granules, powder, or oral liquid formulations such as suspensions or syrups). Commonly used pharmaceutical adjuvants, for example, binders (syrup, gum arabic, gelatin, sorbit, tragacanth, polyvinylpyrrolidone, hydroxypropylcellulose, and such), excipients (lactose, sugar, corn starch, calcium phosphate, sorbit, glycine, and such), lubricants (magnesium stearate, talc, polyethyleneglycol, silica, and such), disintegrants (potato starch, carboxymethylcellulose, and such), moisturizers (sodium laurylsulfate and such) can be used as carriers. Flavors such as strawberry and peppermint can be also added. Moreover, tablets can be coated by common methods. Liquid oral drugs can be solutions or can be used as dried products. Such liquid oral drugs can contain commonly used additives, for example, preservatives (methyl or propyl p-hydroxybenzoate, sorbic acid, and such).

The amount of active component in the pharmaceutical agents can be adjusted according to the extent of the disease and administration method, however, it is usually between 5 and 100% (w/w), and preferably between 10 and 60% (w/w).

The therapeutic methods in the present invention comprise administering a compound, which is an active ingredient of an above described anti-neurodegenerative agent, to a patient who has a neurodegenerative disease. Specifically, the therapeutic methods in the present invention are methods for administering the above described anti-neurodegenerative drugs into patients. Anti-neurodegenerative agents can be administered parenterally (local infusion, intraperitoneal administration, selective intravenous infusion, intravenous injection, subcutaneous injection, organ perfusate infusion, rectal administration, and such) or orally. Moreover, the dose of the pharmaceutical agents varies depending on the age, weight, and symptoms of the patient and the route of administration; however, the amount of the active ingredient can be approximately between 1 and 500 mg/kg.

### Examples

Details of the Examples in the present invention are described below. However, the mode for carrying out the present invention is not to be construed as being limited thereto.

### [Example 1]

(1) 30 µl/ml of solution containing a candidate compound (10 mM, final concentration of 300 µM) was added to RPMI 1640 medium containing THP-1 cells (ATCC TIB202 strain obtained from American Type Culture Collection; 1x 10⁶ cells/ml) and 10% fetal bovine serum (FBS). 100 µl/well of the above mixture was aliquoted into each well of a 24 well plate and incubated for three days at 37°C. The cells were collected, and then lysed in 150 µl of sample lysis solution (composition: 50 mM Tris-HCl (pH 7.5), 150 mM NaCl, and 1% NP-40, into which proteinase inhibitor cocktail (product name: Complete, Roche Diagnostics Corporation) was added according to the attached protocol). The sample solution was recovered by centrifuging the above mentioned cell lysate at 14,000 x g, 4°C for 15 minutes to remove insoluble matter.
(2) Using the sample solution obtained in (1), NAIP concentration in the sample solution was measured using ELISA, according to the method described in Examples 3 and 4 of JP-A 2000-125861. Specifically, 50 µl of the above described sample solution was added to each well of a plate with immobilized anti-NAIP monoclonal antibody. The immobilized antibody used was anti-NAIP antibody hnmc 841, derived from FERM BP-6921 strain (International Patent Organism Depositary accession number, the National Institute of Advanced Industrial Science and Technology (Central 6, 1-1-1 Higashi, Tsukuba, Ibaraki, Japan); deposited on October 9, 1999) which was a hybridoma producing anti-NAIP monoclonal antibody. Measurements were taken three times for each candidate compound, and average values were calculated. As a control (untreated standard), ELISA was also performed on a sample solution processed as for (1), except that an equal amount of distilled water was added in place of the candidate compound solution. Results showed that the sample solution with compound 1 as a candidate compound contained one to three times more NAIP than the untreated standard.

### [Example 2]

(1) 3 ml/well of DMEM-HG medium containing H-SY5Y cells (ATCC CRL 2266 strain obtained from American Type Culture Collection; 1x10⁵ cells/ml), 10% fetal bovine serum (FBS), 100 units/ml penicillin, and 100 µg/ml streptomycin was aliquoted into each well of a 6 well plate, and incubated for two days at 37°C. Either 3 µl/well (lane 2) or 15 µl/well (lane 4) of all-trans retinoic acid (2 mM) was then added to each well. Wells were then incubated for three days at 37°C.
(2) Next, 30 µl/ml of candidate compound solution (10 mM) was added to each well and incubated for one day at 37°C. After incubation, each well was washed with 0.25 M NaCl, then filled with 10% trichloroacetic acid (TCA) on ice, and kept on ice for ten minutes. Cells were collected with a scraper and centrifuged (14,000 rpm, ten minutes, 4°C). To prepare the samples for electrophoresis, 100 µl of urea TX solution (9 M urea solution containing 2% Triton X-100 and 5% 2-mercaptoethanol), 25 µl of 10% lithium dodecyl sulfate (LDS) solution, and 2 µl of 2M Tris solution were added to the obtained pellets. The samples were stored at -80°C.
(3) The samples for electrophoresis obtained in (2) were homogenized and then 10 µl of each sample was loaded onto READYGELSJ (Bio Rad Laboratories) and electrophoresed at 120 V for 75 minutes to conduct SDS-PAGE. After electrophoresis, the protein bands were transferred to a Squi-Blot PVDF Membrane (Bio Rad Laboratories) at 100 V for 75 minutes. The membrane was blocked for one day with TBST (Tris buffered saline solution, pH 7.4 containing 0.05% Tween-20 (Sigma)) containing 10% skim milk, and then washed with TBST. Primary antibody ME1-3 (prepared according to the method described in JP-A Hei 11-116599 and JP-A 2000-125861, identical to biotinylated secondary antibody described in Example 3 (2) in JP-A 2000-125861) was diluted 3000 fold with TBST. The above membrane was immersed in the above primary antibody for two hours at room temperature, washed with TBST, and then immersed for one hour at room temperature in anti-rabbit Ig, horseradish peroxidase (product name; Amersham Pharmacia) that was diluted 3000 fold with TBST. The membrane was developed using a chemiluminescence reagent (ECL-Plus, Amersham Pharmacia) and exposed onto the X-ray film (product name "Bio Max", Kodak) for a 30 minute exposure time. Furthermore, as a control, analysis was also conducted on samples for electrophoresis prepared in the same way as described above in (1) and (2), except a candidate compound was not added (lanes 1 and 3). Moreover, as a comparison, electrophoresis of THP-1 cell contents was conducted in the same way (lane 5). Fig. 1 shows the results. When compound 1 was used as a candidate compound, samples treated with either low or high concentrations of retinoic acid (lanes 2 and 4) expressed significantly strong 160 kDa bands, presumably derived from NAIP, compared to the controls (lanes 1 and 3).

### [Example 3]

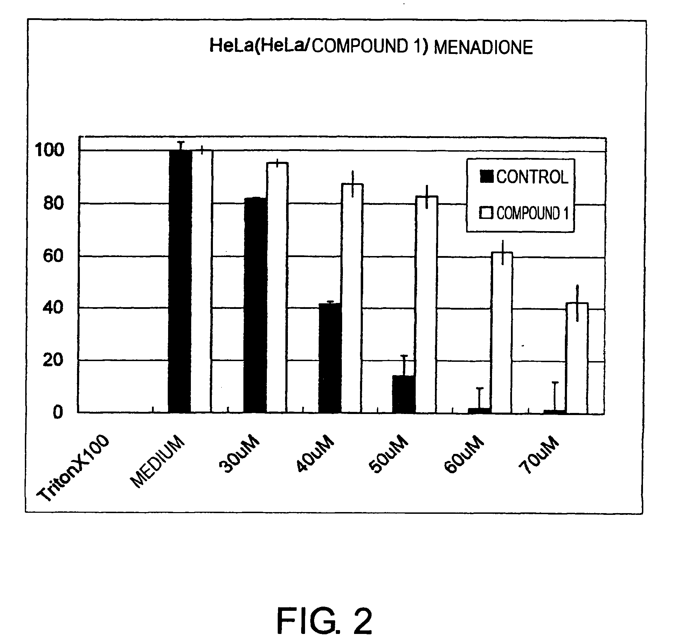

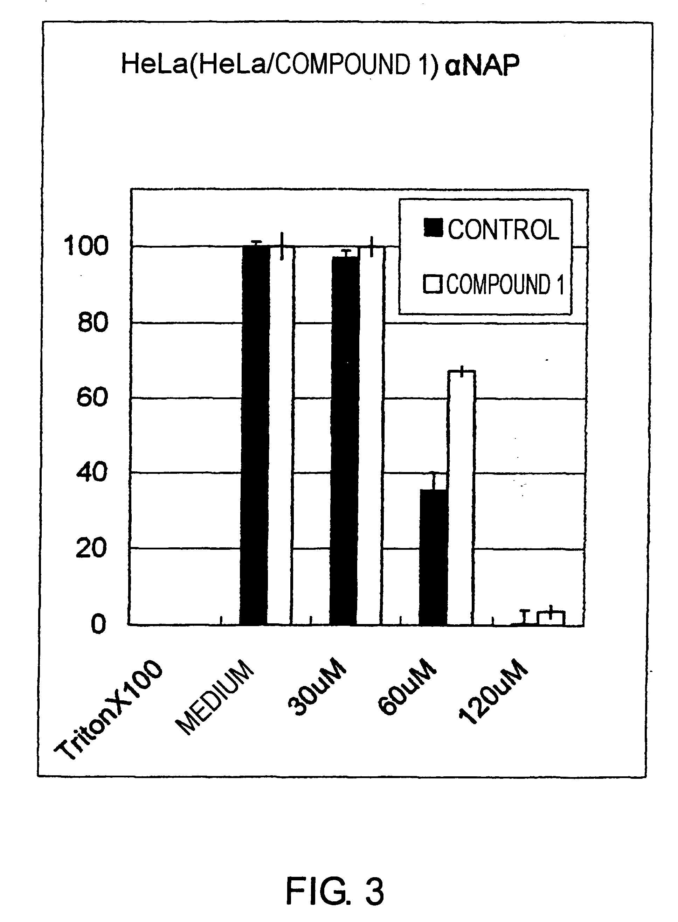

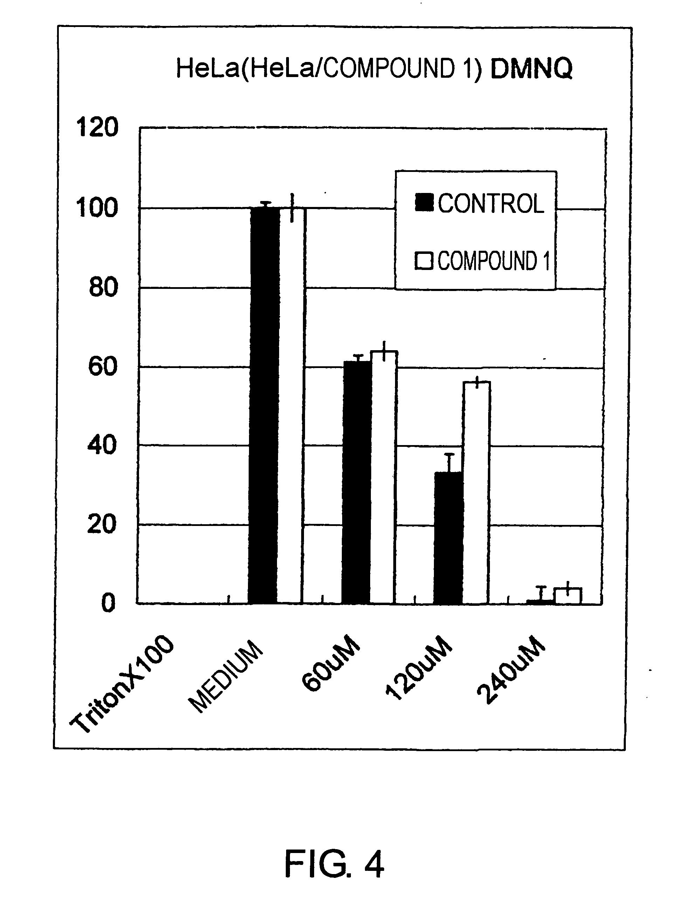

(1) HeLa cells (ATCC CCL2 strain obtained from American Type Culture Collection; 1x 10⁶ cells/ml) were incubated for ten hours at 37°C under 5% CO₂ in DMEM-HG medium supplemented with 10% FBS, 100 units/ml penicillin, and 100 µg/ml streptomycin in a T75 flask A 250 µl solution containing a candidate compound (10 mM) was added to 25 ml of the above culture and incubated for an additional 1.5 days at 37°C under 5% CO₂. Cells were recovered, washed with the above medium, and counted. The cells were resuspended in the above fresh medium so the medium contained 1x 10⁵ cells/ml.
(2) 150 µl of the suspension obtained in (1) was added into each well of a 96 well plate and the plate was incubated for four hours at 37°C. A cell-death inducing agent (any one of menadione (sodium bisulfite, 10 mM solution), α-naphthoquinone (10 mM DMSO solution), or 2, 3-dimethoxy-1, 4- naphthoquinone (10 mM DMSO solution)) was added to each well and the plate was incubated for four hours at 34°C. Cells were washed with the above medium and 150 µl of the above fresh medium was added to each well and incubated for additional six hours at 37°C. 15 µl ofAlamar Blue (product name, BioSource International, Inc. (USA)) was added into each well. After ten hours, luminescence was measured with a luminescence detector to determine the viable cell count at excitation and detection wavelengths of 530 nm and 560 nm, respectively. Moreover, the viable cell count of the control, which was prepared in the same way except that a candidate compound solution was not added, was also determined. An experiment to examine how the presence or absence of a candidate compound affected the correlation between the concentration of cell-death inducing agent and viable cell count was conducted by gradually changing the concentration of cell-death inducing agent. Figs. 2 to 4 show the results when compound 1 was used as a candidate compound. The values shown on the horizontal axis indicate the concentration of cell-death inducing agent. The values on the vertical axis indicate relative fluorescence, where zero defines the fluorescence value obtained from a sample prepared in the same way except that 15 µl of 10% Triton X-100 solution was added instead of cell-death inducing agent. "Control" and "compound 1" show results obtained when using the control and compound 1, respectively.

Table 1 shows some of the results obtained by the experiment in the Examples using various candidate compounds. Furthermore, the experiment shown in Table 1 was conducted using a final concentration of 10 µM of each compound at the time of incubation.

**Table 1**

| Compound number ¹⁾ | Result | Note |
|---|---|---|
| 1061 | ± | Quinpirole, D2-like agonist |
| 0782 | ± | D4 receptor ligand |
| 1005 | ++ | Compound 3, D4 antagonist |
| 1004 | + or ± | Compound 2, D4 antagonist |
| 1065 | ++ | Compound 4, D4 agonist |
| 1002 | ++++ | Compound 1, D4 antagonist |
| 1003 | ± | D2 antagonist |
| 0937 | ± | Pimozide, D2-like antagonist |
| 0524 | ± | D2-like receptor ligand |
| 0701 | ± | 3'-fluorobenzylspiperone, D2-like receptor ligand |

| | | |
|---|---|---|
| 1) Tocris Cookson Ltd. product number. | | |

### [Example 4]

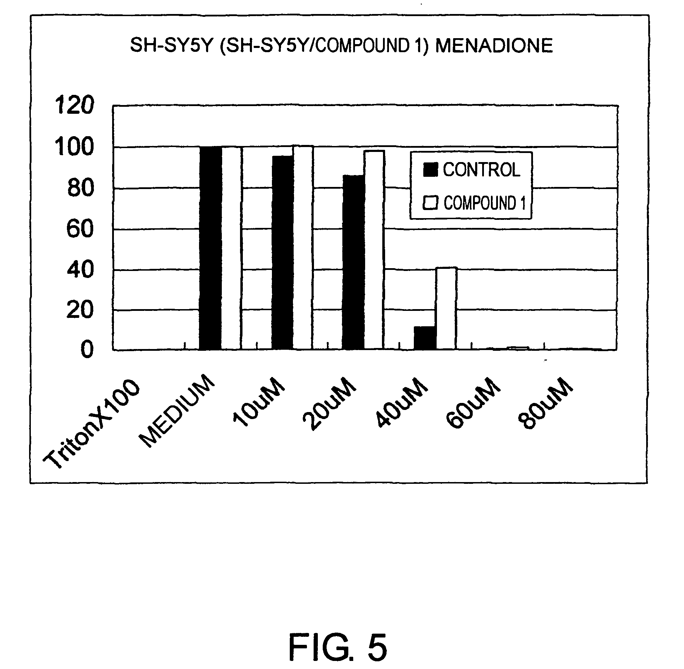

3 ml/well of DMEM-HG medium containing SH-SY5Y cells (1x10⁵ cells/ml), 10% FBS, 100 units/ml penicillin, and 100 µg/ml streptomycin was aliquoted into each well of a 6 well plate. The plate was incubated for two days at 37°C and then 15 µl/well of all-trans retinoic acid solution (2 mM) was added into each well. The plate was incubated for three days at 37°C and then 30 µl/ml of a candidate compound solution (10 mM) was added to each well. The plate was incubated for one day at 37°C. The cells were recovered and washed with the above medium. Cell number was counted and the cells were resuspended in the above fresh medium at 1x 10⁵ cells/ml. This suspension was processed as for that in Example 3 (2), to examine how the presence or absence of a candidate compound affected the correlation between the concentration of cell-death inducing agent and viable cell count. Fig. 5 shows the results obtained when compound 1 was used as a candidate compound and menadione was used as cell-death inducing agent.

### [Example 5]

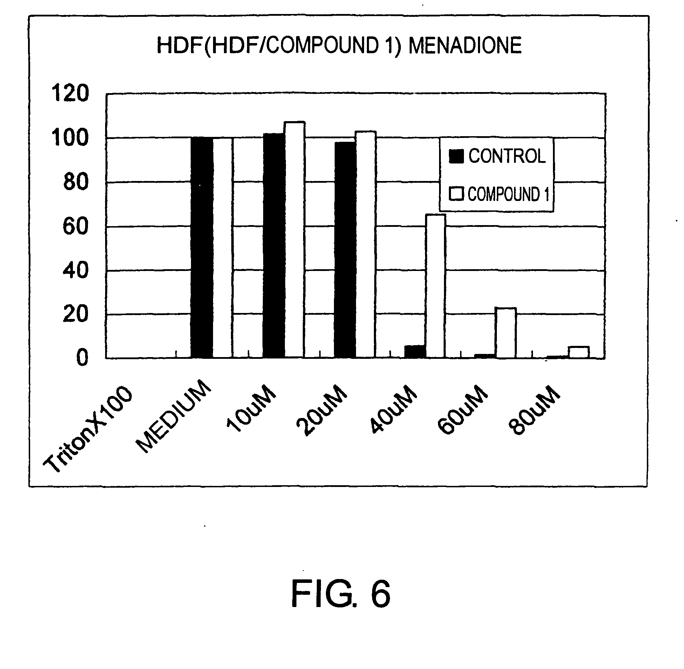

Human fibroblast cells (catalog # 106-05) contained in a normal human fibroblast cell culture kit from Cell Applications, Inc., instead of HeLa cells, were processed as in Example 3 to examine how the presence or absence of a candidate compound affected the correlation between the concentration of cell-death inducing agent and viable cell count. Fig 6 shows the results obtained when compound 1 was used as a candidate compound and menadione was used as a cell-death inducing agent.

### [Example 6-1~6-4]

A candidate compound was dissolved in physiological saline solution to prepare a 100 mM solution with the pH adjusted to 3 to 4 with 1 N NaOH as necessary. This solution was stored. The solution was diluted with physiological saline at time of use to prepare 0.5 ml of a solution containing 8 mg (Example 6-1), 40 mg (Example 6-2), 80 mg (Example 6-3), or 240 mg (Example 6-4) of a candidate compound. The above solution was administered to Mongolian gerbils. In Examples 6-1 to 6-3, 0.5 ml of the above solution was orally administered every 24 hours. In Example 6-4 the solutions was administered once. Furthermore, abnormalities caused by administration were not observed and blood pressure, temperature, and electrocardiograph monitors all showed normal, except for the appearance of catalepsy-like symptoms when the above solution containing 240 mg of a candidate compound was administered twice.

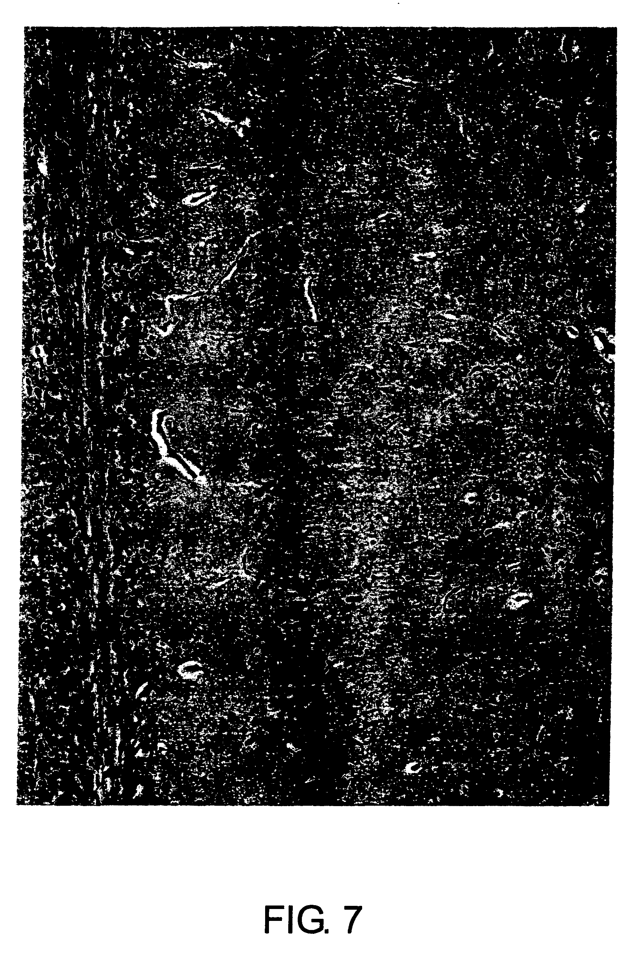

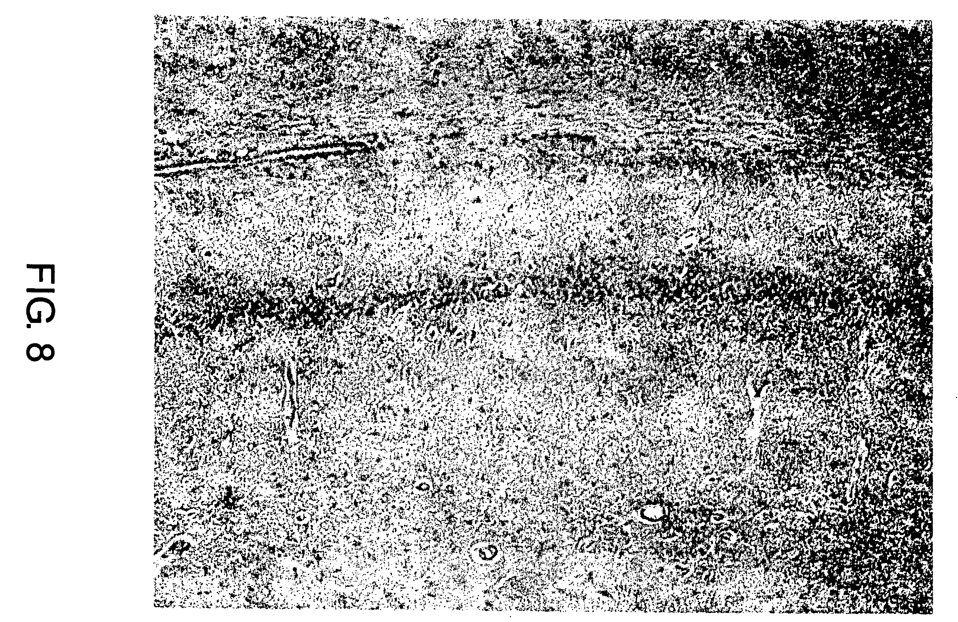

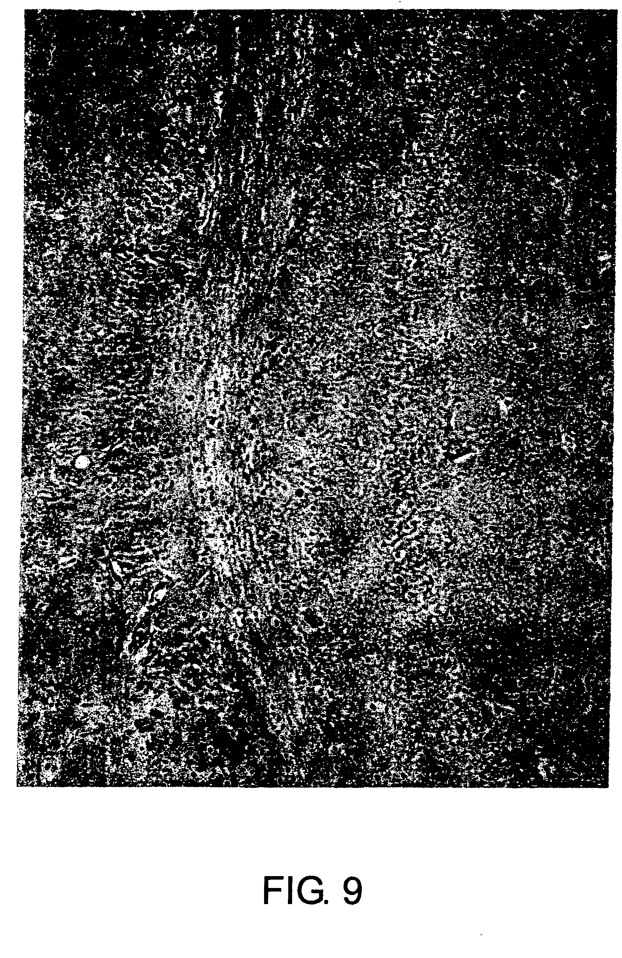

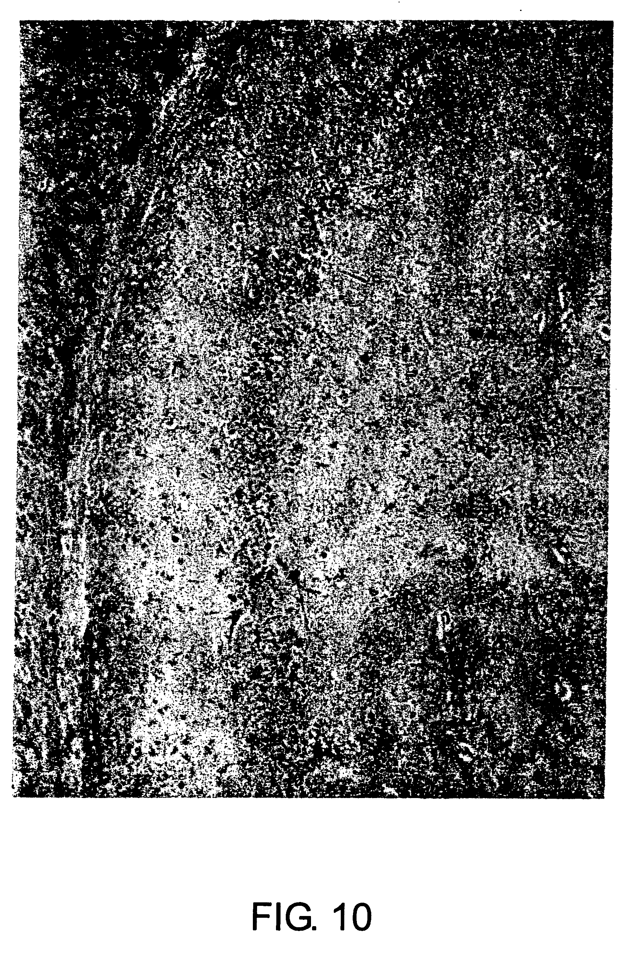

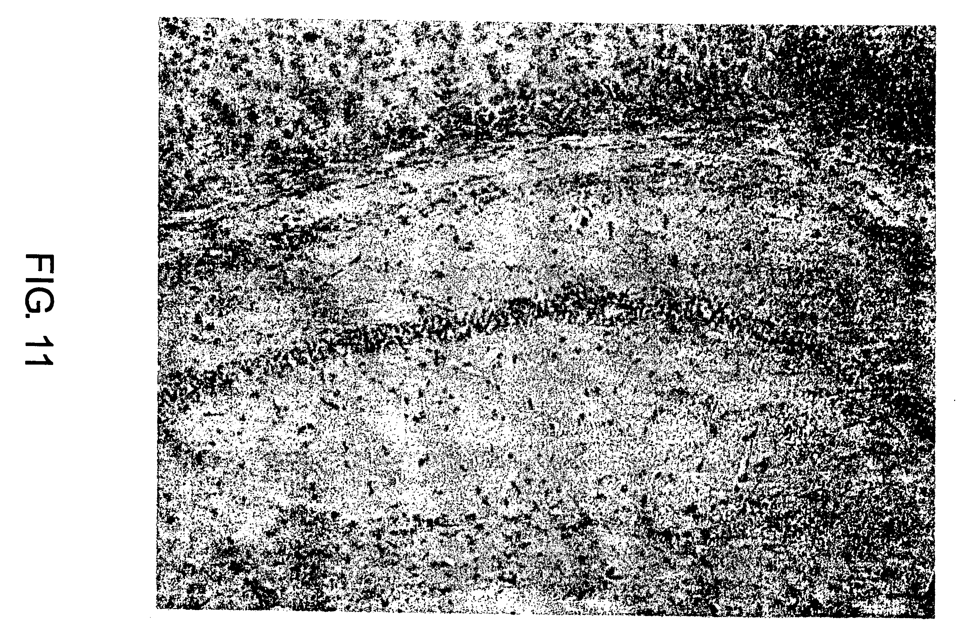

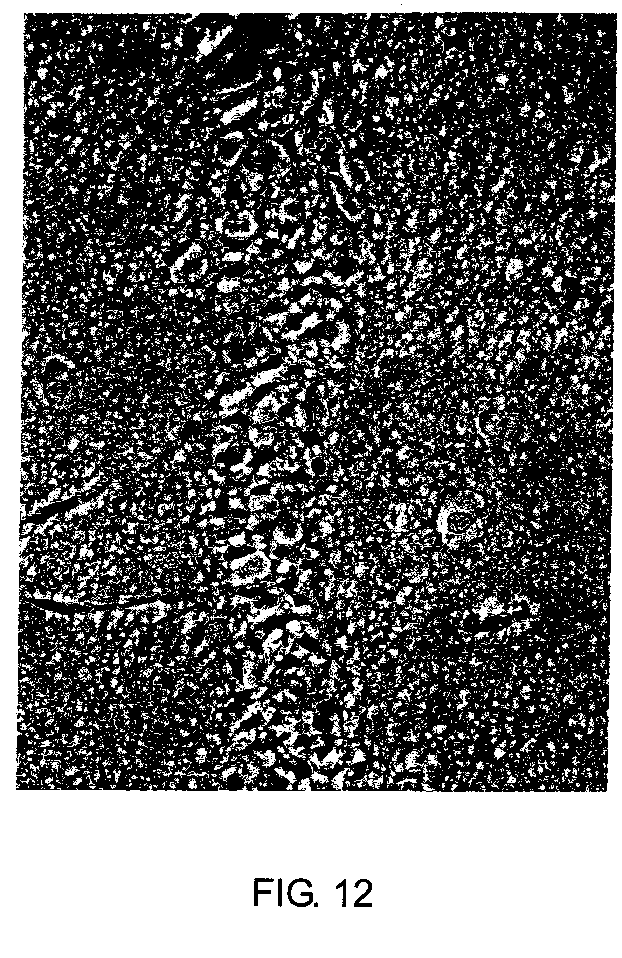

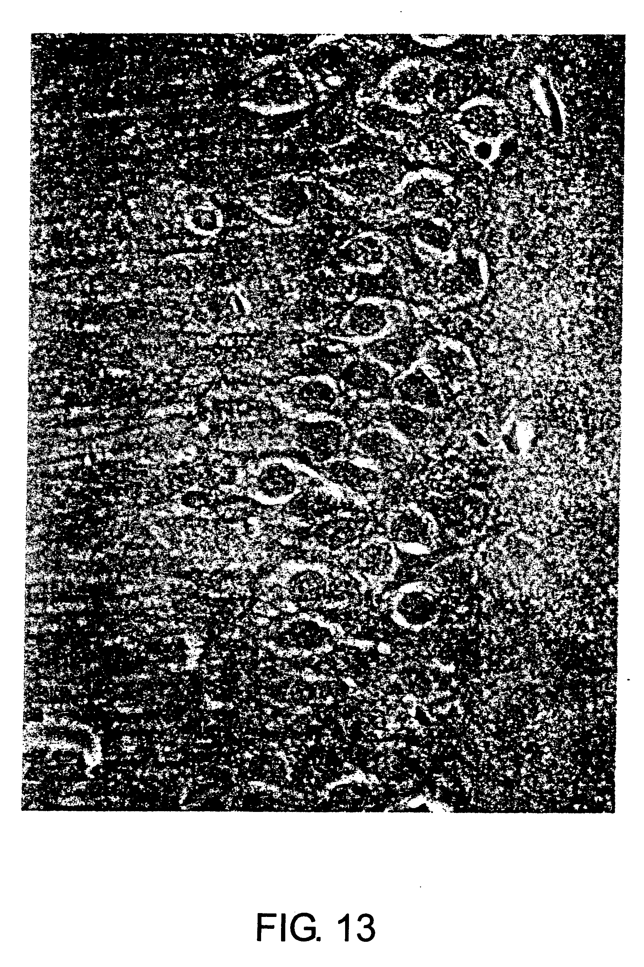

Two hours after the second administration in Examples 6-1 to 6-3, both Mongolian gerbil common carotid arteries were occluded for ten minutes. The gerbils were sacrificed five days later. Two hours after administration in Example 6-4, both Mongolian gerbil common carotid arteries were occluded for ten minutes, and the gerbils were sacrificed three days later. Hematoxylin-eosin stained cross-sections of the brain hippocampus CA1 region tissue were prepared, and the stained sections were examined. Moreover, Mongolian gerbils in which the same process was performed, except that administration of a compound and occlusion were not performed, and other gerbils in which the same process was performed, except that the administration of a compound was not performed, were also sacrificed, and cross-sections were prepared in the same way and used as controls. Figs. 7 to 13 show the results obtained when compound 1 was used as a candidate compound and the results of the control. As shown in Figs. 7 to 13, dose-dependent suppression of cell deformation and deciduation in the CA1 region was observed when compound 1 was used as a candidate compound.

### [Example 7]

The *in vivo* therapeutic effect of compound 1 was examined using an Amyotrophic Lateral Sclerosis (ALS) model animal.

A Cu/Zn-SOD gene transgenic mouse (Saishin Igaku, Vol. 57 (7), "new Amyotrophic Lateral Sclerosis (ALS) model animals", July, 2002: p.1622-1627: obtained from Dr. Masashi Aoki at Tohoku University, School of Medicine, Neurology) was used. Three ALS model mice were divided into three groups (physiological saline administered group, compound 1 (8 mg/kg) administered group, compound 1 (40 mg/kg) administered group; one mouse each). Physiological saline, compound 1 (8 mg/kg), and compound 1 (40 mg/kg) were orally administered to each ALS mouse once a week from about 40 days before the predicted day of the symptom development (140-150 days after birth) until the day the mice died.

The results are shown in Table 2, and it was confirmed that administration of compound 1 (40 mg/kg) had an effect in prolonging the period between the development of symptoms and the death of the ALS model mouse.

**Table 2**

| Treatment | Days before symptom development | Days of survival | Days between symptom development and death |
|---|---|---|---|
| Physiological saline | 145 | 196 | 24 |
| Compound 1 (8 mg/kg) | 144 | 196 | 25 |
| Compound 1 (40 mg/kg) | 146 | 177 | 31 |

### Industrial Applicability

As described above in detail, when administered to subjects, the anti-neurodegenerative agents of the present invention comprise the effect of increasing NAIP production, and further of suppressing neurodegeneration. Therefore, the anti-neurodegenerative agents of the present invention are useful for treating and preventing neurodegenerative diseases such as amyotrophic lateral sclerosis (ALS), spinal muscular atrophy (SMA), Huntington's disease, Parkinson's disease, Alzheimer's disease, cerebrospinal paralysis accompanied by injury and cerebral vascular disorder, dementia after cerebral vascular disorder, and dementia accompanied by other neuronal degeneration.

## Claims

1. An anti-neurodegenerative agent comprising one, two or more compounds selected from the group consisting of 3-[4-(4-chlorophenyl)piperazin-1-yl]methyl]-1H-pyrrolo[2,3-b] pyridine or salts thereof, 5-(4-chlorophenyl)-4-methyl-3-(1-(2-phenylethyl) piperidin-4-yl) isoxazole or salts thereof, 3-(4-chlorophenyl)-4-methyl-5-(1-(2-phenylethyl) piperidin-4-yl) isoxazole or salts thereof, and N-methyl-4-(2-cyanophenyl) piperazinyl-3-methylbenzamine or salts thereof.

2. A method for producing an anti-neurodegenerative agent using one, two or more compounds selected from the group consisting of 3-[4-(4-chlorophenyl) piperazin-1-yl]methyl]-1H-pyrrolo [2,3-b] pyridine or salts thereof, 5-(4-chlorophenyl)-4-methyl-3-(1-(2-phenylethyl) piperidin-4-yl) isoxazole or salts thereof, 3-(4-chlorophenyl)-4-methyl-5-(1-(2-phenylethyl) piperidin-4-yl) isoxazole or salts thereof, and N-methyl-4-(2-cyanophenyl) piperazinyl-3-methylbenzamine or salts thereof.

3. A method for treating a neurodegenerative disease comprising administering one, two or more compounds selected from the group consisting of3-[4-(4-chlorophenyl) piperazin-1-yl] methyl]-1H-pyrrolo [2,3-b] pyridine or salts thereof, 5-(4-chlorophenyl)-4-methyl-3-(1-(2-phenylethyl) piperidin-4-yl) isoxazole or salts thereof, 3-(4-chlorophenyl)-4-methyl-5-(1-(2-phenylethyl) piperidin-4-yl) isoxazole or salts thereof, and N-methyl-4-(2-cyanophenyl) piperazinyl-3-methylbenzamine or salts thereof to a patient who has a neurodegenerative disease.
